# EUROPEAN PATENT APPLICATION

(11) **EP 4 613 844 A1**
(43) Date of publication of application: **10.09.2025**
(21) Application number: 25162140.5
(22) Date of filing: 06.03.2025
(51) Int. Cl.: C12N 1/14, C12Q 1/04

(54) **GROWTH MEDIUM FOR THE DETECTION OF FILAMENTOUS FUNGI AND USE THEREOF**

(30) Priority: 06.03.2024 NL 2037185
(71) Applicant: Royal Lactalis Leerdammer B.V., 4145 KK Schoonrewoerd (NL)
(72) Inventor: Sakidin, Chadi Sunario, 4145 KK Schoonrewoerd (NL); Vermeulen, Dewi, 4145 KK Schoonrewoerd (NL); Sebus, Johannes Frederik, 4145 KK Schoonrewoerd (NL)
(74) Representative: Cabinet Le Guen Maillet

(57) **Abstract**

The invention pertains to a growth medium composition selective for filamentous fungi, its use in quality control and/or for detecting filamentous fungi in a sample, a method for detecting filamentous fungi in a sample and a kit-of-parts to prepare the growth medium composition.

## Description

### Field of the Invention

The invention is in the field of growth media for fungi and mould, and detection methods for filamentous fungi.

### Background

Filamentous fungi are a class of fungi characterised by cells that grow as tubular, elongated, and thread-like. Mould is the growth of mycelium of hyphae (vegetative filaments) of filamentous fungi.

There is a high interest in detecting filamentous fungi that form mould in a variety of samples. For example, spores of filamentous fungi may be airborne and present in ventilation or air conditioning conducts. Certain types of mould are toxic to humans. Mould is also known to spoil foods, and this affects food production processes as well as food quality. The presence of mould may result in spoilt batches of food products, which need to be recalled or destroyed. Not only undetected filamentous fungi may lead to significant food waste, but it also poses health and safety concerns. In view of these concerns the presence of mould in food is highly monitored in quality control procedures. Therefore, there is a need to detect the presence of filamentous fungi in various steps of food production before mould starts growing.

Traditionally mould and yeast are monitored together. Detection is performed by placing a diluted sample on an agar plate, commonly Malt Extract Agar, allowing for an incubation period, counting the number of colony forming units (CFUs) and calculating the concentration based on the dilution of the sample. An example of such method is reported by Kure et al. in the context of monitoring of mould in cheese production processes (Kure and Skaar "The fungal problem in cheese industry" Current Opinion in Food Science 2019, 29: 14-19).

CN109929903A provides a growth medium for detecting mould and yeast in a sample.

US2008261229A1 provides a method for detecting simultaneously multiple microbes' classes (bacteria, mould and yeast).

Kabir et al. ("Isolation of pectinase producing bacteria from the Rhiszosphere of Andrographis paniculata Nees and 16S rRNA gene sequence comparison of some potential strains" Advances in microbiology, 2019, 09: 1 - 13) mentions a medium for screening pectinase activity, wherein the medium comprises 1.5% agar and 0.2% pectin. The pectin is not further defined. Jayasankar et al. ("An agar plate method for screening and enumerating pectinolytic microorganisms" Canadian journal of microbiology 1970, 16: 1023) provides a gel medium comprising 2% agar, 0,5% pectin and 0.1% yeast extract for screening pectinase activity. A demethylated pectin derived from apple is used.

CN104031839 concerns a method for screening rotten fungi on the surface of mulberry using a culture medium comprising agar, pectin and ammonium sulphate. The pectin is not defined.

Bezawada et al. ("Screening of pectinolyticfungi and optimization of process parameters using guava peel powder as substrate under solid state fermentation" IJESI 2018, 7: 43-47) provides a gel medium to isolate pectinolytic fungi. The medium comprising 1% pectin, 2% agar and 0.14% ammonium sulphate. The pectin is not defined.

Amilia et al. ("Isolation and screening of pectinolytic fungi from orange (Citrus nobilis Tan.) and banana (Musa acuminata L.) fruit peel" lOP Conf Ser: Mater. Sci Eng. 2017, 193: 012015) mentions a method to isolate or screen for pectinolytic fungi from orange or banana peel. The method includes using a gel medium comprising 0.5% pectin, 2% agar and 0.1% yeast extract. Pectin is not further defined.

Nevertheless, detecting the presence of filamentous fungi is particularly challenging when the environment comprises other microorganisms, in particular yeast. Yeast may outcompete mould in detection assays and make it difficult to count mould CFUs, resulting in an underestimation of the presence of mould. This problem is particularly challenging in samples where there is a large excess of yeast to mould. The above cited methods are not selective for mould and therefore do not provide a solution to this issue.

There is a need in the art for a method to selectively detect filamentous fungi in the context of quality control, especially in environments where other microorganisms, in particular yeast, are present.

### Summary of the Invention

The inventors have surprisingly found that by employing pectin as a source of carbohydrates in an agar-based growth medium, it was possible to grow mould selectively over yeast, thus making it possible to detect mould in high-yeast environments.

Therefore, the invention concerns a growth medium composition comprising carbohydrate, wherein the carbohydrate comprises at least pectin and agar, preferably wherein pectin and agar are the sole carbohydrates. The growth medium composition of the invention may be a liquid or may be a gel. Typically, the growth medium is a liquid composition that then is solidified into a gel.

The invention further concerns the use of a growth medium composition comprising carbohydrates, wherein the carbohydrates comprise pectin and agar, preferably wherein pectin and agar are the sole carbohydrates, for
- quality control, and/or
- detecting filamentous fungi in a sample. The detection of filamentous fungi involves the detection of the formation of moulds that grow in the growth medium composition.

The invention further concerns a method for detecting mould of filamentous fungi in a sample, wherein the method comprises
a. providing the sample to a container, preferably a petri dish, comprising the growth medium composition of the invention;
b. incubating the container at 10 to 40 °C for at least 1 day up to at most 18 days;
c. counting the colony forming units (CFU) of mould.

In a preferred aspect the growth medium composition used in the method is prepared from a liquid composition that then is solidified into a gel.

Finally, the invention concerns a kit of parts comprising
i. powdered carbohydrate, wherein the powdered carbohydrate comprises
   a. powdered pectin, and
   b. powdered agar;
ii. optionally at least one container; and
iii. an instructions manual;
wherein pectin and agar are the sole carbohydrates.
1. A selective growth medium composition comprising carbohydrates, wherein the carbohydrates comprise pectin and agar, preferably wherein pectin and agar are the sole carbohydrates of the growth medium.
2. The growth medium composition according to embodiment 1, comprising pectin in an amount of 0.05 - 10 wt.% based on total weight of the composition.
3. The growth medium composition according to embodiment 1 or 2, wherein the amount of pectin and agar exceeds the amount of any monosaccharide or disaccharide by 50% based on the total weight of monosaccharides and disaccharides.
4. The growth medium composition according to any one of the preceding embodiments, wherein the pectin to agar weight ratio is in the range of 25:1 to 1:90, preferably 10:1 to 1:60, more preferably 10:1 to 1:10, most preferably 6:1 to 1:1.
5. The growth medium composition according to any one of the preceding embodiments, wherein the growth medium is for the detection of filamentous fungi, preferably wherein the growth medium is selective for the growth of filamentous fungi.
6. The growth medium composition according to any one of the preceding embodiments, wherein the composition further comprises a nitrogen source, wherein preferably the nitrogen source is creatine, at least an ammonium salt, at least a nitrate salt, or combinations thereof, more preferably at least a nitrate salt.
7. The growth medium composition according to any one of the preceding embodiments, wherein the growth medium composition is a gel.
8. Use of the gel growth medium composition according to embodiment 7, for quality control.
9. Use according to embodiment 8, wherein quality control is of food preparation processes and/or of air conditioning of buildings.
10. Use of the gel growth medium composition according to embodiment 7, for detecting filamentous fungi in a sample, wherein the sample preferably further comprises yeast.
11. A method for detecting filamentous fungi in a sample, wherein the method comprises
   a. providing the sample to a container, preferably a petri dish, comprising the gel growth medium composition of claim 7;
   b. incubating the container at a temperature of 10 to 40 °C, preferably 20 to 30 °C, more preferably 23 to 28 °C for at least 1 day up to at most 18 days;
   c. counting the colony forming units (CFU) of mould.
12. The use according to embodiment 10 or the method according to embodiment 11, wherein the sample further comprises yeast, preferably wherein the yeast is selected from the subdivision *Saccharomycotina,* more preferably from *Ascoidea, Babjeviella, Brettanomyces, Candida, Clavispora, Cyberlindnera, Debaryomyces, Diutina, Eremothecium, Hyphopichia, Kazachstania, Kluyveromyces, Komagataella, Kuraishia, Lachancea, Lodderomyces, Metschnikowia, Meyerozyma, Naumovozyma, Ogataea, Pichia, Saccharomyces, Saccharomycodes, Saprochaete, Scheffersomyces, Spathaspora, Sugiyamaella, Suhomyces, Tetrapisispora, Torulaspora, Wickerhamiella, Wickerhamomyces, Yamadazyma, Yarrowia, Zygosaccharomyces, Zygotorulaspora* or combinations thereof.
13. The use according to embodiment 10 or the method according to embodiment 11, wherein the sample is selected from brine, a food product, or air, preferably wherein the sample is a food product, wherein the food product is selected from milk, cheese, and yogurt.
14. The growth medium composition of embodiment 5, the use of embodiment 10 or the method according to embodiment 11, wherein the filamentous fungi is selected from the genus *Penicillium* and/or *Aspergillus,* more preferably from *Aspergillus niger, Penicillium chrysogenum, Penicillium crustosum, Penicillium expansum, Penicillium verrucosum, Penicillium roqueforti, Penicillium commune,* or combinations thereof.
15. A kit of parts comprising
   i. powdered carbohydrate, wherein the powdered carbohydrate comprises
      a. powdered pectin, and
      b. powdered agar;
   ii. optionally at least one container; and
   iii. an instructions manual;
   wherein pectin and agar are the sole carbohydrates.

### Brief description of the figures

**Figure 1** shows petri dishes containing MEA (top) and FFA (bottom) growth media inoculated with 50.000 yeast cells (5×10⁴ cfu *D. hansenii)* and 25 spores (25 cfu *P. crustosum)* at 3 (left) and 13 (right) days of incubation.
**Figure 2** shows the average recovery of filamentous fungi in the presence of increased yeast population. The x axis shows the excess of yeast to mould in ratio of mould to yeast or log difference. A log difference of 0 means no yeast present, values > 0 represent log factors of yeast competition: a log value of 1 represents a 10 fold excess of yeast, a log value of 2 100 fold excess of yeast, and so on. The y axis shows the colony forming units (CFUs) recovered of filamentous fungi mould.
**Figure 3** shows the growth of mould on a regular MEA plate (left) for a brine sample at 10x dilution and the growth of mould on a FFA plate (right) for a brine sample at 0x dilution.
**Figure 4** shows the setup of a MAS-100 air sampler.
**Figure 5** shows the growth of microorganisms on a MEA plate (left) and FFA plate (right) for an air sample.

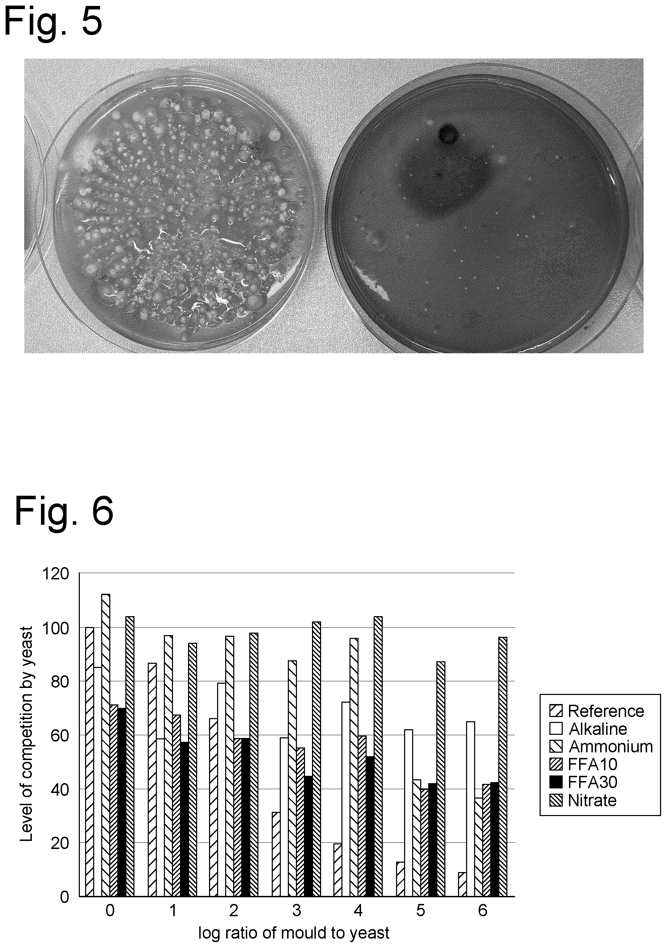

**Figure 6** shows the recovery of filamentous fungi mould in the presence of increased yeast population on different growth media. The x axis shows the level of competition by yeast, expressed as excess of yeast to mould as log difference. A log ratio of 0 means no yeast present, values > 0 represent log factors of yeast competition, a log value of 1 represents a 10-fold excess of yeast, a log value of 2 100-fold excess of yeast, and so on. The y axis shows the indexed mould recovery, i.e. colony forming units (CFUs) of filamentous mould on test agar with or without yeast, compared to the CFUs on reference agar without yeast, which was set at 100.
**Figure 7** shows the recovery of filamentous fungi at different pectin concentrations in the absence or presence of yeast. The x axis shows the pectin concentration in the growth medium (g/L), while the y axis shows the colony forming units (CFUs) recovered of filamentous fungi mould.

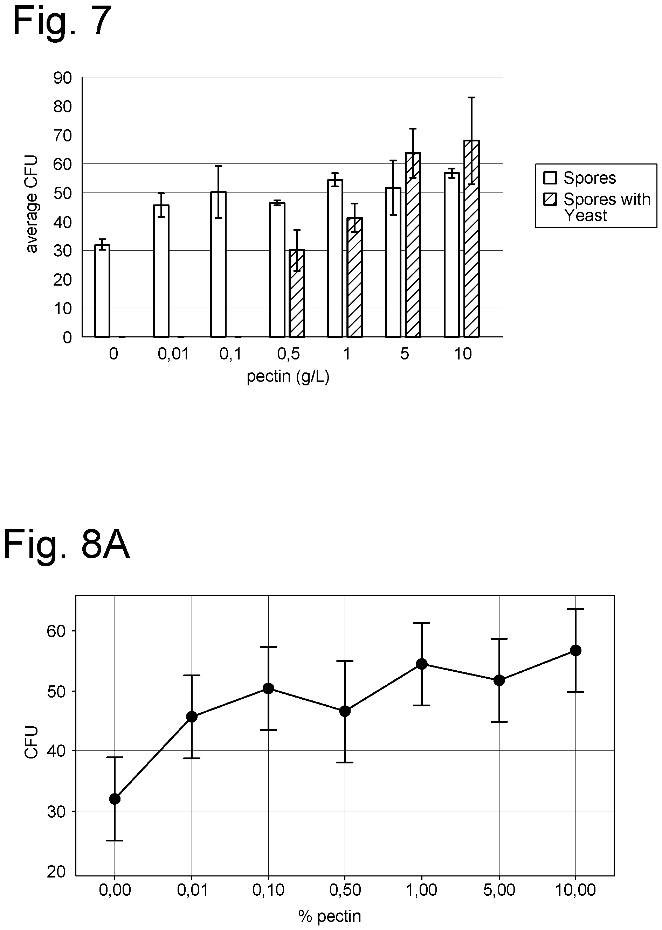

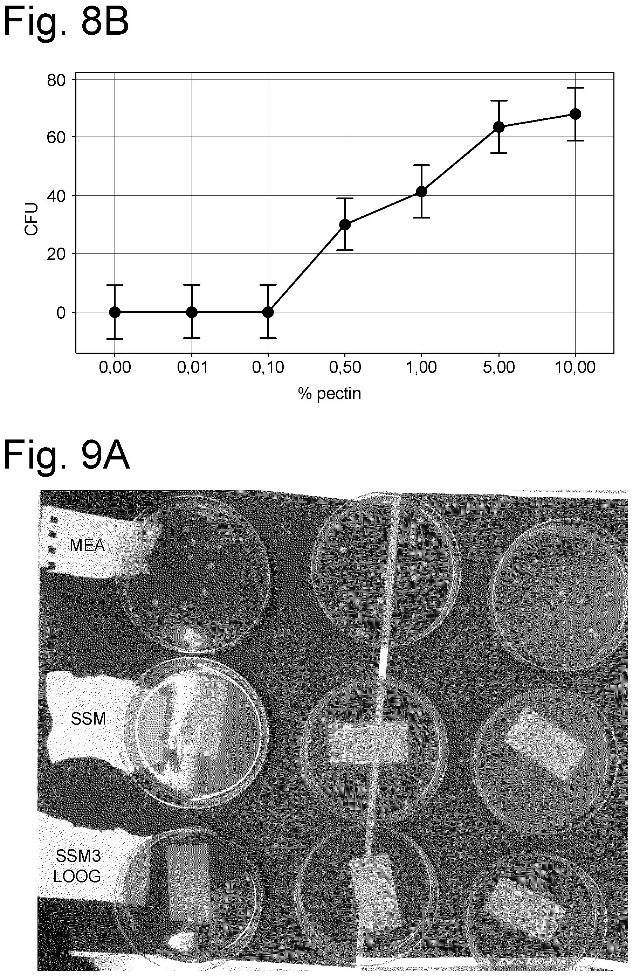

**Figure 8A** **and** **8B** shows the statistical analysis of the recovery of filamentous fungi (y axis) at different pectin concentrations in the medium (x axis) when the sample contains only spores of filamentous fungi (Figure 8A) or spores of both filamentous fungi and yeast (Figure 8B). The bars represent 95% confidence intervals based on the pooled standard deviation of the data. An overlap in confidence intervals implies that there is no statistical difference between the two datapoints (p>0.05). When two confidence intervals do not overlap, they are statistically different datapoints (p<0.05).
**Figure 9A** **and** **9B** show colonies of yeasts on agar plates. The yeasts are *Kluyveromyces lactis* (Figure 9A) and *Debaryomyces hansenii* (Figure 9B). The top row plates correspond to the reference medium Malt Extract Agar (MEA), while the middle and bottom row plates correspond to the media of the invention.

### Detailed description

The inventors have found that a growth medium composition comprising pectin and agar as sole carbohydrates was ideal for the growth of mould. The growth medium compositions were surprisingly found to be selective for mould, in particular selective over yeast.

Without wishing to be bound to a theory, it is believed that the presence of pectin promotes selective growth of mould over yeast. Most of the common agar media such as those based on Malt Extract Agar (MEA), Potato Dextrose Agar (PDA), dichloran rose Bengal chloramphenicol agar (DRBC), dichloran 18% glycerol agar (DG18), are normally composed of simple sugars (mono- or di-saccharides) which can be metabolised by both yeast and moulds. Generally, yeast grows more quickly than moulds and is thus able to inhibit mould growth and impairs detection thereof. On the other hand, pectin is a complex polysaccharide composed mostly of galacturonic acid units, which may be functionalised with acetylated hydroxyl and methylated carboxyl groups. Given the complex molecular structure of pectin, microorganisms require specialized enzymes for the degradation of this molecule.

The ability to degrade pectin is not common in yeast. Even if yeast show some ability to degrade pectin, they do not harbour the full spectrum of enzymes needed to effectively consume the substrate (Alimardani-Theuil et al. "Yeasts: An attractive source of pectinases-From gene expression to potential applications: A review." Process Biochem. 2011, 46: 1525-1537; Biely and Sláviková "New search for pectolytic yeasts. " Folia Microbiol. (Praha) 1994, 39: 485-488; Dasilva et al. "Pectinolytic enzymes secreted by yeasts from tropical fruits. " FEMS Yeast Res. 2005, 5: 859-865; Schwan et al. "Endopolygalacturonase secretion by Kluyveromyces marxianus and other cocoa pulp-degrading yeasts. " Enzyme Microb. Technol. 1997, 21: 234-244).

In contrast, filamentous fungi have the ability to degrade pectin. *Penicillium roqueforti* is used in biotechnology industry because of its ability to produce pectinolytic enzymes and a wide variety of other hydrolytic proteins (Mioso et al. "Penicillium roqueforti: a multifunctional cell factory of high value-added molecules. " J. Appl. Microbiol. 2015, 118: 781-791). *Aspergillus fungi* are also able to produce a wide variety of hydrolytic enzymes, including 3 types of pectinolytic enzymes (Acuña-Argüelles et al. "Production and properties of three pectinolytic activities produced byAspergillus niger in submerged and solid-state fermentation. " Appl. Microbiol. Biotechnol. 1995, 43: 808-814). Filamentous fungi possess a more extensive array of hydrolytic enzymes compared to yeasts.

Consequently, the invention concerns a growth medium composition, the use of such growth medium composition for quality control and/or for detecting mould and/or filamentous fungi in a sample, a method for detecting mould in a sample and a kit-of-parts for preparing the growth medium composition. All aspects and embodiments of the invention are described below in detail. Those skilled in the art will understand that the features described in connection to the growth medium composition are applicable to the use, method, and kit of parts of the invention.

### Definitions

The term "filamentous fungi" as used herein refers to fungi having cells that grow as tubular, elongate, and thread-like structures called hyphae, which may contain multiple nuclei and extend by growing at their tips.

The term "mould" as used herein refers to a non-taxonomic grouping of fungi that grow in the form of mycelium of hyphae (vegetative filaments). The terms "mould" and "mold" are synonyms. It is noted that the term "filamentous fungi" as used herein refers to the fungal microorganism, while the term "mould" as used herein refers to the visible growth of vegetative filaments. The term "mould-forming filamentous fungi" refers to filamentous fungi that are able to form mould. Since all filamentous fungi may form mould, "filamentous fungi" and "mould-forming filamentous fungi" are synonyms.

The term "pectin" as used herein refers to the heteropolysaccharides contained in the primary cell wall and intracellular layer of plant cells, mainly in fruits such as apples, oranges, and lemons. The principal, chemical component of pectin is galacturonic acid (a sugar acid derived from galactose), while pectin comprises also sub-units based on arabinose.

The term "agar" as used herein refers to a mixture of two polysaccharides: agarose and agaropectin. Agarose is a linear polymer, containing repeating units of agarobiose, a disaccharide comprising units of D-galactose and 3,6-anhydro-L-galactopyranose. Agaropectin is a heterogeneous mixture of smaller molecules that occur in lesser amounts, and is made up of alternating units of D-galactose and L-galactose, functionalised with acidic side-groups, such as sulphate, glucuronate, and pyruvate.

The term "growth medium composition" as used herein refers to a composition providing nutrients used for growing microorganisms and detecting them.

The term "selective" as used herein in the context of a growth medium refers to a growth medium containing ingredients that supress the growth of certain microorganisms in favour of others. In the context of the present invention the selective growth medium favors growth of filamentous fungi over moult.

The term "quality control" as used herein refers to a procedure or set of procedures intended to ensure that a manufactured product or system adheres to a defined set of quality criteria.

The term "food preparation process" as used herein refers to any process aimed at preparing a food or drink product.

The term "gel" as used herein refers to a semi-solid, which is a dilute cross-linked system exhibiting no flow when in the steady state, although the liquid phase may diffuse through the cross-linked system.

All percentages are by weight unless otherwise stated.

In addition, in the context of the invention, the terms "comprising" or "comprises" do not exclude other possible elements. The product of the present invention, including the many embodiments described herein, can comprise, consist of, or consist essentially of the essential elements and limitations of the invention described herein, as well as any additional or optional ingredients, components, or limitations described herein or otherwise depending on the needs. Any reference to prior art documents in this specification is not to be considered an admission that such prior art is widely known or forms part of the common general knowledge in the field.

The invention will now be described in further details. It is noted that the various aspects, features, examples and embodiments described in the present application may be compatible and/or combined together.

### Growth medium composition

In a first aspect, the invention concerns a growth medium composition comprising carbohydrate, wherein the carbohydrate comprises pectin and agar, preferably wherein pectin and agar are the sole carbohydrates. With sole carbohydrates it is meant that the sum of pectin and agar is at least 80 wt.%, preferably at least 90 wt.%, more preferably at least 95 wt.%, most preferably at least 98 wt.% of the total carbohydrate.

Preferably, the growth medium composition promotes colony formulation and/or growth of mould of filamentous fungi. Preferably, the growth medium composition is selective for filamentous fungi. More preferably, the growth medium composition is selective for filamentous fungi in the presence of yeast. More preferably, the growth medium composition promotes the colony formation and/or growth of mould of filamentous fungi in the presence of yeast. Preferably the growth medium composition is a selective growth medium composition selective for the growth of filamentous fungi. Preferably, the filamentous fungi are selected from the genus *Penicillium* and/or *Aspergillus,* more preferably from *Aspergillus niger, Geotrichum candidum, Penicillium chrysogenum, Penicillium crustosum, Penicillium expansum, Penicillium verrucosum, Penicillium roqueforti, Penicillium commune,* or combinations thereof. Preferably, yeast is selected from the subdivision *Saccharomycotina,* more preferably from *Ascoidea, Babjeviella, Brettanomyces, Candida, Clavispora, Cyberlindnera, Debaryomyces, Diutina, Eremothecium, Hyphopichia, Kazachstania, Kluyveromyces, Komagataella, Kuraishia, Lachancea, Lodderomyces, Metschnikowia, Meyerozyma, Naumovozyma, Ogataea, Pichia, Saccharomyces, Saccharomycodes, Saprochaete, Scheffersomyces, Spathaspora, Sugiyamaella, Suhomyces, Tetrapisispora, Torulaspora, Wickerhamiella, Wickerhamomyces, Yamadazyma, Yarrowia, Zygosaccharomyces, Zygotorulaspora* or combinations thereof.

The growth medium composition of the invention may be in any suitable form. Preferably, the composition is a liquid or a gel, preferably a gel. Typically, the growth medium composition is prepared as a liquid and then solidified into a gel. More preferably the composition is a gel.

### Pectin and Agar

Pectin may be obtained from any source known to the skilled person. Pectin contains high esterification or low esterification. Preferably the pectin has an esterification of 50 to 90%, more preferably 60 to 80%. Preferably the pectin is citrus derived. An example of a suitable pectin is CAS number 9000-69-5, which is a citrus derived pectin, with a degree of esterification of about 67 to 71 %.

Agar may be obtained from any source known to the skilled person.

Pectin and agar are in a preferred aspect the sole carbohydrates in the growth medium of the composition, meaning that the sum of pectin and agar is at least 80 wt.%, preferably at least 90 wt.%, more preferably at least 95 wt.%, most preferably at least 98 wt.% of the total carbohydrate. Preferably the amount of pectin is at least 90 wt.%, more preferably at least 95 wt.%, most preferably at least 98 wt.% of the carbohydrate discounting for agar. Discounting for agar as used herein refers to the carbohydrates present with the exception of agar, i.e. apart from agar. Preferably the amount of pectin is at least 30 wt.%, more preferably at least 40 wt.%, most preferably at least 50 wt.% of the total carbohydrate accounting for agar.

Preferably the amount of pectin and agar exceeds the amount of any monosaccharide or disaccharide by 50%, more preferably by 70%, most preferably by 100% based on the total weight of monosaccharides and disaccharides. Most preferably, the growth medium composition is free of monosaccharides and disaccharides.

Preferably the growth medium composition comprises pectin in an amount of 0.05 - 10 wt.%, more preferably 0.1 - 5 wt.%, more preferably 0.2 - 3 wt.%, most preferably 0.5 - 1 wt.% based on total weight of the composition. Preferably the growth medium composition comprises pectin in an amount of 5 - 70 g per kg of composition, more preferably 10 - 50 g per kg of composition, most preferably 20 - 40 g per kg of composition. Due to pectin's low water solubility and difficulties in processing composition comprising pectin, it is preferred that the amount of pectin is at most 10 wt.%, more preferably at most 5 wt.%, most preferably at most 3 wt.% based on total weight of the composition.

Preferably the growth medium composition comprises agar in an amount of 0.5 - 10 wt.%, more preferably, 0.5 - 5 wt.%, most preferably 1 - 4 wt.% based on total weight of the composition. Preferably the growth medium composition comprises agar in an amount of 1 - 60 g per kg of composition, more preferably 5 - 40 g per kg of composition, most preferably 10 - 30 g per kg of composition.

The weight ratio of pectin to agar in the growth medium composition is preferably in the range of 25:1 to 1:90, preferably 10:1 to 1:60, more preferably 10:1 to 1:10, more preferably 8:1 to 1:2, most preferably 6:1 to 1:1

In a further preferred aspect, the weight ratio of pectin to agar in the growth medium composition is in the range of 6:1 to 1:1, preferably 4:1 to 1:1, more preferably 3:1 to 1.2:1, even more preferably 2:1 to 1.2:1, most preferably about 1.5:1.

### Nitrogen source

The growth medium composition of the invention preferably comprises a nitrogen source. Preferably the nitrogen source is selected from creatine, an ammonium salt, a nitrate salt or combinations thereof. More preferably the nitrogen source is creatine, a nitrate salt or combinations thereof, most preferably the nitrogen source is at least a nitrate salt.

Preferably the growth medium composition comprises creatine in an amount of 0.01 - 5 wt.%, more preferably 0.05 - 2 wt.%, most preferably 0.1 - 0.5 wt.% based on total weight of the composition. Preferably the growth medium composition comprises creatine in an amount of 0.1 - 15 g per kg of composition, more preferably 1 - 10 g per kg of composition, most preferably 1 - 5 g per kg of composition.

Preferably the growth medium composition comprises at least a nitrate salt, such as sodium nitrate, potassium nitrate, calcium nitrate, zinc nitrate, copper nitrate, ammonium nitrate, and combinations thereof, in an amount of 0.01 - 5 wt.%, more preferably 0.05 - 3 wt.%, most preferably 0.1 - 1.5 wt.% based on total weight of the composition. Preferably the growth medium composition comprises at least a nitrate salt, such as sodium nitrate, potassium nitrate, calcium nitrate, zinc nitrate, copper nitrate, ammonium nitrate, and combinations thereof, in an amount of 0.1 - 20 g per kg of composition, more preferably 1 - 15 g per kg of composition, most preferably 2 - 10 g per kg of composition.

In a further embodiment, preferably the growth medium composition comprises at least an ammonium salt, such as ammonium sulphate, ammonium chloride, ammonium phosphate, ammonium nitrate, and combination thereof, in an amount of 0.01 - 5 wt.%, more preferably 0.05 - 2 wt.%, most preferably 0.1 - 1 wt.% based on total weight of the composition. Preferably the growth medium composition comprises at least an ammonium salt, such as ammonium sulphate, ammonium chloride, ammonium phosphate, ammonium nitrate, and combinations thereof, in an amount of 0.1 - 20 g per kg of composition, more preferably 1 - 15 g per kg of composition, most preferably 2 - 10 g per kg of composition.

### Optional components

The growth medium composition of the invention preferably comprises further components. The composition may comprise any component commonly used in growth media. Preferably, the composition further comprises chloramphenicol, dichloran, bromocresol purple, or combinations thereof.

Preferably the growth medium composition comprises chloramphenicol in an amount of 0.001 - 0.2 wt.%, more preferably 0.001 - 0.1 wt.%, most preferably 0.001 - 0.05 wt.% based on total weight of the composition. Preferably the growth medium composition comprises chloramphenicol in an amount of 1 - 100 mg per kg of composition, more preferably 10 - 80 mg per kg of composition, most preferably 30 - 70 mg per kg of composition. In an alternative preferred embodiment the composition does not comprise chloramphenicol.

Preferably the growth medium composition comprises oxytetracycline and/or dichloran, preferably dichloran in an amount of 0.0001 - 0.01 wt.%, more preferably 0.0001 - 0.001 wt.%, most preferably 0.0001 - 0.0005 wt.% based on total weight of the composition. Preferably the growth medium composition comprises dichloran in an amount of 0.05 - 20 mg per kg of composition, more preferably 0.1 - 10 mg per kg of composition, most preferably 1 - 5 mg per kg of composition.

Preferably the growth medium composition comprises bromocresol purple in an amount of 0.001 - 0.2 wt.%, more preferably 0.001 - 0.1 wt.%, most preferably 0.001 - 0.05 wt.% based on total weight of the composition. Preferably the growth medium composition comprises bromocresol purple in an amount of 1 - 100 mg per kg of composition, more preferably 10 - 80 mg per kg of composition, most preferably 30 - 70 mg per kg of composition.

### Salts

Preferably the growth medium composition further comprises inorganic salts. Preferably the inorganic salts are selected from chlorides, phosphates, sulphates, and combinations thereof. Preferably the inorganic salts are salts of potassium, magnesium, zinc, copper, or sodium. More preferably the inorganic salts are selected from the list consisting of potassium chloride, potassium phosphate, potassium sulphate, magnesium chloride, magnesium phosphate, magnesium sulphate, zinc chloride, zinc phosphate, zinc sulphate, copper chloride, copper phosphate, copper sulphate, sodium chloride, sodium phosphate, sodium sulphate, or combinations thereof. Most preferably the inorganic salts are selected from potassium chloride, potassium phosphate, magnesium sulphate, zinc sulphate, copper sulphate, or combinations thereof.

Preferably the growth medium composition comprises inorganic salts in an amount of 0.005 - 2 wt.%, more preferably 0.01 - 1 wt.%, most preferably 0.05 - 0.5 wt.% based on total weight of the composition. Preferably the growth medium composition comprises inorganic salt in an amount of 0.05 - 7 g per kg of composition, more preferably 0.1 - 5 g per kg of composition, most preferably 0.5 - 3 g per kg of composition.

### Use

The invention concerns the use of the growth medium composition described above for quality control. Preferably the use according to the invention is for quality control of air ventilation systems or air conditioning systems of a building. Preferably the use according to the invention is for quality control of a food preparation process. Preferably the food preparation process is selected from preparation of alcoholic drinks, beer brewing, preparation of wine, preparation of soft fermented drinks like kombucha, preparation of fruit juices, preparation of dairy-type products such as milk, yogurt, and cheese, preparation of protein plant based products, such as soy, pulse, rice based products, preparation of kettle food, preparation of cereal based products or fermented cereal based products, preparation of baked goods, preparation of fresh wheat products, such as pasta, preparation of rice based products, such as para-boiled rice or rice puddings. More preferably the process is selected from a process wherein high level of yeast are present, for example processes where brine is used. More preferably the process is selected from beer brewery, preparation of baked goods, or dairy products such as milk, cheese, and yogurt preparation. Most preferably the process is cheese preparation.

In a preferred embodiment, the use in quality control involves
- retrieval of a sample from a food preparation process as described above, and
- testing of the sample for the presence of filamentous fungi.

The invention concerns the use of the growth medium composition according to the invention for detecting filamentous fungi in a sample.

Preferably the filamentous fungi to be detected are selected from the genus *Penicillium* and/or *Aspergillus,* more preferably from *Aspergillus niger, Geotrichum candidum, Penicillium chrysogenum, Penicillium crustosum, Penicillium expansum, Penicillium verrucosum, Penicillium roqueforti, Penicillium commune,* or combinations thereof.

The use according to the invention is particularly preferred for samples derived from environments wherein yeast is also present, wherein said yeast strains are preferably selected from the subdivision *Saccharomycotina,* more preferably from *Ascoidea, Babjeviella, Brettanomyces, Candida, Clavispora, Cyberlindnera, Debaryomyces, Diutina, Eremothecium, Hyphopichia, Kazachstania, Kluyveromyces, Komagataella, Kuraishia, Lachancea, Lodderomyces, Metschnikowia, Meyerozyma, Naumovozyma, Ogataea, Pichia, Saccharomyces, Saccharomycodes, Saprochaete, Scheffersomyces, Spathaspora, Sugiyamaella, Suhomyces, Tetrapisispora, Torulaspora, Wickerhamiella, Wickerhamomyces, Yamadazyma, Yarrowia, Zygosaccharomyces, Zygotorulaspora* or combinations thereof.

Preferably, the sample is derived from air conditioning system or air ventilation system of a building. Preferably the sample is derived from a food preparation process as described above. The sample may be derived from any stage of the production chain. Preferably the sample is selected from brine, a food product or air. More preferably the sample is a food product. In a preferred embodiment the food product is selected from a dairy product, more preferably from milk, cheese, and yogurt, most preferably cheese. In another preferred embodiment the food product is a baked goods, such as bread. Most preferably the sample comprises yeast.

### Method

The invention pertains to a method for detecting filamentous fungi in a sample, wherein the method comprises
a. providing the sample to a container, preferably a petri dish, comprising the solidified growth medium composition of the invention;
b. incubating the container at 10 to 40 °C, preferably 20 to 30 °C, more preferably 23 to 28 °C for at least 1 day up to at most 18 days;
c. counting the colony forming units (CFU) of mould.

Thanks to the method according to the invention, it is possible to detect filamentous fungi in any environment wherein mould may cause issues. The method may also be employed to detect filamentous fungi in environments wherein mould is a desired end-product, for example in the preparation of matured cheeses. The method may be used to detect filamentous fungi in any sample that may contain such fungi.

Preferably, the filamentous fungi to be detected are selected from the genus *Penicillium* and/or *Aspergillus,* more preferably from *Aspergillus niger, Geotrichum candidum, Penicillium chrysogenum, Penicillium crustosum, Penicillium expansum, Penicillium verrucosum, Penicillium roqueforti, Penicillium commune,* or combinations thereof.

The method is particularly preferred for samples derived from environments wherein yeast is also present, wherein yeast is preferably selected from the subdivision *Saccharomycotina,* more preferably from *Ascoidea, Babjeviella, Brettanomyces, Candida, Clavispora, Cyberlindnera, Debaryomyces, Diutina, Eremothecium, Hyphopichia, Kazachstania, Kluyveromyces, Komagataella, Kuraishia, Lachancea, Lodderomyces, Metschnikowia, Meyerozyma, Naumovozyma, Ogataea, Pichia, Saccharomyces, Saccharomycodes, Saprochaete, Scheffersomyces, Spathaspora, Sugiyamaella, Suhomyces, Tetrapisispora, Torulaspora, Wickerhamiella, Wickerhamomyces, Yamadazyma, Yarrowia, Zygosaccharomyces, Zygotorulaspora,* or combinations thereof.

Preferably the sample is derived from air conditioning system or air ventilation system of a building. Preferably the sample is derived from a food preparation process. Preferably the food preparation process is selected from preparation of alcoholic drinks, beer brewing, preparation of wine, preparation of soft fermented drinks like kombucha, preparation of fruit juices, preparation of dairy products such as milk, yogurt, and cheese, preparation of dairy-like plant based products, such as soy, pulse, rice based products, preparation of kettle food, preparation of cereal based products or fermented cereal based products, preparation of baked goods, preparation of fresh wheat products, such as pasta, preparation of rice based products, such as para-boiled rice or rice puddings. More preferably the process is selected from a process wherein high level of yeast are present, for example processes wherein brine is used. More preferably the process is selected from beer brewery, preparation of baked goods, or dairy products, such as milk, cheese, and yogurt, preparation. Most preferably the process is cheese preparation.

The sample may be derived from any stage of the production chain. Preferably the sample is selected from brine, a food product or air. More preferably the sample is a food product. In a preferred embodiment the food product is selected from a dairy product, more preferably milk, cheese, and yogurt, most preferably cheese. In another preferred embodiment the food product is a baked goods, such as bread. Most preferably the sample comprises yeast.

Preferably the sample is provided undiluted to the container comprising the growth medium composition of the invention.

Preferably the sample is incubated at a temperature of 10 - 40 °C, more preferably 20 - 30 °C, most preferably 23 - 28 °C. The sample is incubated for at least 1 day and up to a maximum of 18 days. Preferably the sample is incubated for 2 - 14 days, more preferably 3 - 10 days.

### Kit of parts

The invention concerns a kit of parts comprising
i. powdered carbohydrate, wherein the powdered carbohydrate comprises
   a. powdered pectin, and
   b. powdered agar;
ii. optionally at least one container; and
iii. an instructions manual;
wherein pectin and agar are the sole carbohydrates.

With sole carbohydrate it is meant that the sum of pectin and agar is at least 80 wt.%, preferably at least 90 wt.%, more preferably at least 95 wt.%, most preferably at least 98 wt.% of the total carbohydrate.

Preferably the amount of pectin is at least 90 wt.%, more preferably at least 95 wt.%, most preferably at least 98 wt.% of the carbohydrate discounting for agar. Preferably the amount of pectin is at least 30 wt.%, more preferably at least 40 wt.%, most preferably at least 50 wt.% of the total carbohydrate accounting for agar.

Preferably the amount of pectin and agar exceeds the amount of any monosaccharide or disaccharide by 50%, more preferably by 70%, most preferably by 100% based on the total weight of monosaccharides ad disaccharides. Most preferably, the carbohydrate of the kit of parts of the invention is free of monosaccharides and disaccharides.

Preferably, the weight ratio of pectin to agar in the kit-of parts is in the range of 25:1 to 1:90, 10:1 to 1:60, preferably 10:1 to 1:10, preferably 6:1 to 1:1, preferably 4:1 to 1:1, more preferably 3:1 to 1.2:1, even more preferably 2:1 to 1.2:1, most preferably about 1.5:1.

Preferably the container is at least one petri dish.

The kit of parts preferably further comprises optional ingredients in powdered form. Preferably, the optional ingredients are selected from creatine, chloramphenicol, dichloran, bromocresol purple, inorganic salts or combinations thereof, more preferably at least inorganic salts.

The skilled person will understand that the kit of parts according to the invention can be readily used to prepare the growth medium composition of the invention and to carry out the use and method of the invention as described above.

### Examples

### Example 1 - Preparation of the growth medium composition

In this example the growth medium composition of the invention (filamentous fungi agar or FFA) is prepared. The recipe for FFA used in Example 1 is reported in Table 1.

**Table 1. Recipe of FFA in Example 1.**

| **Component** | **Amount (g)** |
|---|---|
| Pectin | 30 |
| Agar | 20 |
| Creatine | 3.0 |
| KCl | 0.5 |
| K₂HPO₄ | 1.0 |
| MgSO₄ × 7 H₂O | 0.5 |
| ZnSO₄ × 7 H₂O | 0.01 |
| CuSO₄ × 5 H₂O | 0.005 |
| Chloramphenicol | 0.05 |
| Dichloran | 0.002 |
| Bromocresol purple | 0.05 |
| Water to fill | 1000 |

FFA is prepared as follows. Pectin and agar are weighted out on a scale. 90 vol.% of water based on desired final volume of the composition is heated until boiling. Pectin and agar are dissolved in the boiling liquid under strong stirring. Additional ingredients are weighted out on the scale. Additional ingredients are dissolved in the remaining 10 vol.% of water based on the desired final volume. After pectin, agar, and all additional ingredients are dissolved, the solutions are combined and allowed to cool to about 60 °C. The pH is adjusted to around 4.5-5 by adding HCl or NaOH. The pH of the final composition of Table 1 is 4.82. The hot liquid is distributed in small bottles. The small bottles are sterilised by autoclave at 121 °C for 20 minutes.

### Example 2 - growth of moulds on a growth medium control according to prior art (MEA) and the growth medium of the invention (FFA)

In this example the growth of mould of filamentous fungi on the growth medium of the invention (Filamentous Fungi Agar or FFA) was tested and compared to the growth of mould on a control growth medium known in the art: Malt Extract Agar (MEA).

### Methods

FFA was prepared as in Example 1. MEA was prepared by dissolving 50 g of Malt Extract Agar (OXOID, lot 3612623, of which the composition is 30 gram malt extract, 5 gram mycological peptone, 15 gram agar) in water.

Spore solutions were prepared by first growing the filamentous fungi on MEA agar of 5-10 days at 25 °C followed by storage in fridge at 4 °C for 5-10 days. 4 ml of Ringers solution with 0.05% Tween80 was gently washed over the mycelium, releasing the spore without damaging the hyphae. The Spore solution was removed from the agar plate and counted with under a microscope using a Thoma counting chamber. The solution was then diluted to reach the desired concentration.

Diluted spore solutions were placed on a petri dish containing either FFA or MEA. The dishes were incubated at 25 °C for 5-10 days.

### Results

The results of growth are shown in Table 2.

**Table 2. Recovery of mould spores solution of MEA and FFA.**

| **Scientific name** | **CFU on MEA** | **CFU on FFA** |
|---|---|---|
| *Aspergillus niger* | 4.0 | 3.7 |
| *Geotrichum candidum* | 39.3 | 36.7 |
| *Penicillium chrysogenum* | 4.3 | 3.7 |
| *Penicillium commune* | 18.7 | 17.7 |
| *Penicillium crustosum* | 1.3 | 1.0 |
| *Penicillium expansum* | 1.3 | 1.7 |
| *Penicillium roqueforti* | 1.0 | 1.0 |
| *Penicillium verrucosum* | 36.0 | 24.7 |

These results show that the mould of filamentous fungi could be grown of FFA as well as on MEA. Detection by recovery of CFUs of mould was comparable.

### Example 3 - test in presence of yeast

In this example FFA is tested as a growth medium for mould when yeast is also present in the sample. FFA is compared to a control growth medium known in the art: Malt Extract Agar (MEA).

### Sample preparation

Mixtures of mould spores and yeast culture were prepared in such a way that varying ratio of mould and yeast were obtained. Approximately 25 moulds were present in all samples while decimal increase of yeast were present (e.g. 25 spore vs 25, 250, 2.500, 25.000, 250.000 and 2.500.000 yeast).

### Methods

FFA and MEA were prepared as in Examples 1 and 2. The mixtures comprising mould and yeast were plated on a Petri dish containing either MEA or FFA. The Petri dishes were incubated at 25 °C. The dishes were checked on day 3 and day 13. After incubation, the colony forming units (CFUs) of mould were counted.

### Results

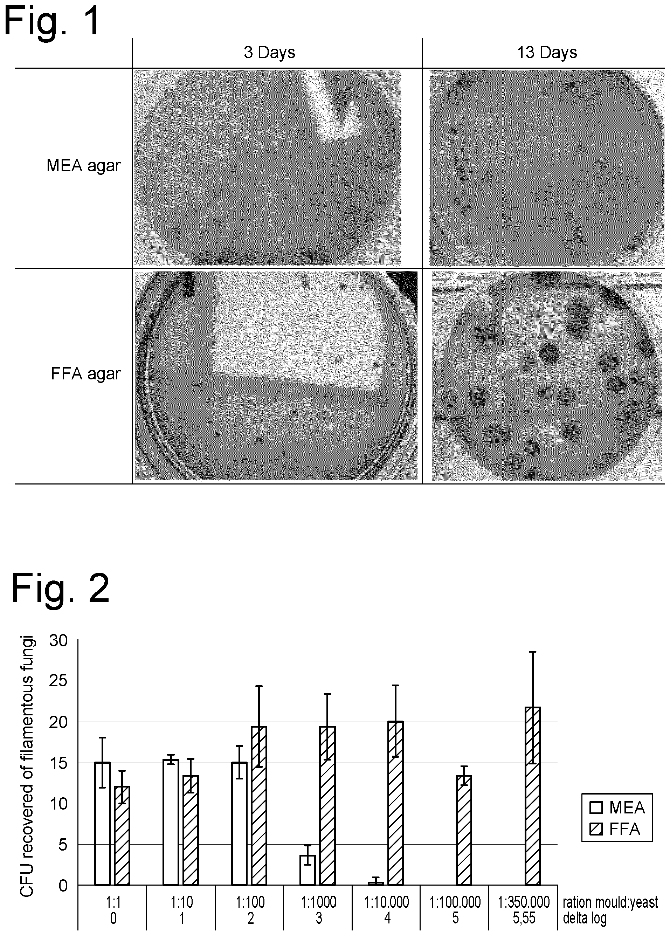

The results are shown in Figures 1 and 2.

On the control growth medium MEA (Figure 1, top) yeast takes over and outnumbers the mould. At high concentration of yeast, germination of *P. crustosum* (mould) is suppressed. At 3 and 13 days it is virtually impossible to count the CFUs of *P. crustosum* on MEA. In contrast, mould of *P. crustosum* is clearly visible on the growth medium of the invention FFA. On FFA it is possible to count the CFUs of mould at both 3 and 13 days even in the presence of high quantities of yeast.

Figure 2 shows the recovery of filamentous fungi in presence of increasing yeast population. When the mould is outnumbered by the yeast in a relatively low fraction (1:1 - 1:100), the mould is still able to develop properly on both MEA and FFA. On the control agar MEA, once the numbers of yeast are higher than 3 log difference the mould is not able to completely develop. At a log difference of log 5, the mould is not able to break through the high amount of yeast and no CFUs of mould can be counted on MEA. In contrast, mould still develops on the growth medium of the invention FFA, regardless of the excess of yeast present. It is possible to count the mould CFUs even at more than difference of log > 5.

In summary, on known growth media (MEA), a high amount of yeast inhibits the germination and growth of mould, resulting in the underestimation of CFUs of mould of filamentous fungi. On FFA it is possible to count CFUs of mould of filamentous fungi even at high concentrations of yeast. These results show that the growth medium of the invention (FFA) is selective for the detection of filamentous fungi.

### Example 4 - test on factory brine

In this example the factory brine of a cheese production process is monitored for presence of filamentous fungi.

### Methods

FFA and MEA were prepared as in Examples 1 and 2. Samples of brine were obtained from the factory brine of a cheese production process. The samples of brine were provided to petri dishes containing either MEA or FFA. Brine was diluted 10x using sterile Ringers solution. The undiluted Brine was plated on FFA while the 10x diluted Brine was plated on MEA. The dishes were incubated at 25 C for 7 days.

### Results

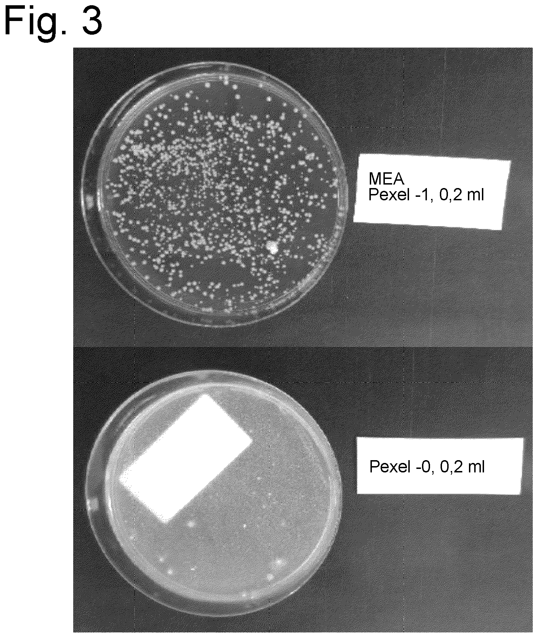

Due to the presence of yeast overgrowing the medium, the samples plated on MEA had to be 10x diluted. Only then it was possible to observe countable CFU of mould (Figure 3). In contrast, the sample did not need any dilution when plated on FFA. Factory brine was takes as is and plated. Since FFA is selective for mould, CFUs of mould were easily countable even at 0x dilution, thereby enabling the detection of fewer quantities of mould in the sample (Figure 3).

### Example 5 - test on factory air

In this example the factory air of a cheese production process is monitored for presence of filamentous fungi.

### Methods

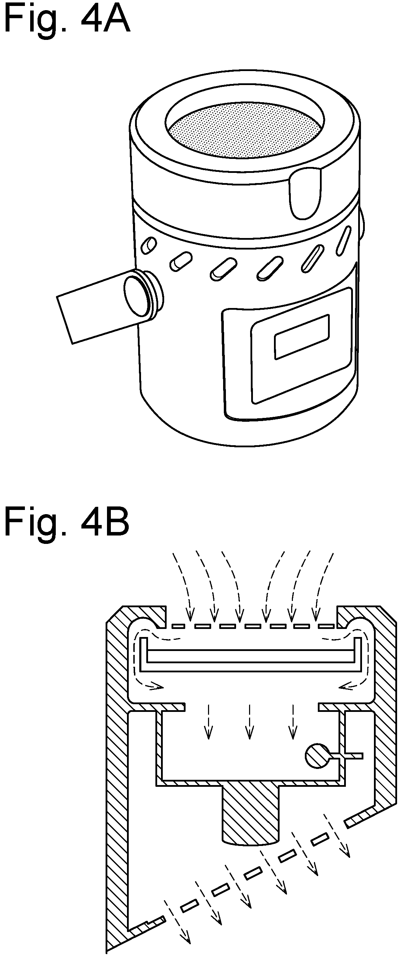

FFA and MEA were prepared as in Examples 1 and 2. Sample of air were taken from the air of a cheese production process by using a MAS-100 air sampler. A petri dish containing either MEA or FFA agar was placed inside the air sampler and 100 litres of air is collected through the perforated top plate (Figure 4). The dishes were incubated at 25 °C for 6 days.

### Results

The factory air may comprise spores of filamentous fungi as well as airborne yeast and other microorganisms deriving from the cheese production process. In principle, all microorganisms may grow on the agar plates. On traditional MEA growth of all microorganisms from the sample is observed (left in Figure 5). The MEA plate is oversaturated causing reduced accuracy of detection of mould of filamentous fungi. In contrast, the FFA plate leads to less growth, mainly of mould (right in Figure 5). This leads to increased visibility and ease of detection of mould of filamentous fungi, i.e. a selective advantage for moulds.

This result corroborated that FFA inhibits and/or reduces the growth of microorganisms other than mould. FFA is a selective growth medium for mould of filamentous fungi.

### Example 6 - different recipes of the growth medium according to the invention

In this example, different recipes for the FFA were prepared.

The different recipes are reported in Table 3. FFA30 comprises more pectin than the other recipes and is similar to FFA of Examples 1-5. FFA-Alkaline and FFA-10 comprise 10 g of pectin and the dissolution of pectin in FFA-Alkaline was performed at high pH. FFA-Nitrate and FFA-Ammonium comprise sodium nitrate and ammonium sulphate, respectively, as nitrogen sources instead of creatine.

**Table 3. Recipes of FFA in Example 6. All amounts are in g.**

| **Component** | **FFA30** | **FFA-Alkaline** | **FFA10** | **FFA-Nitrate** | **FFA-Ammonium** |
|---|---|---|---|---|---|
| Pectin | 30 | 10 | 10 | 10 | 10 |
| Agar | 15 | 20 | 20 | 20 | 20 |
| Creatine | 3.0 | 3.0 | 3.0 | | |
| NaNO₃ | - | - | - | 6 | - |
| (NH₄)₂SO₄ | - | - | - | - | 5 |
| KCl | 0.5 | 0.5 | 0.5 | 0.5 | 0.5 |
| K₂HPO₄ | 1.0 | 1.0 | 1.0 | 1.0 | 1.0 |
| MgSO₄ × 7 H₂O | 0.5 | 0.5 | 0.5 | 0.5 | 0.5 |
| ZnSO₄ × 7 H₂O | 0.01 | 0.01 | 0.01 | 0.01 | 0.01 |
| CuSO₄ × 5 H₂O | 0.005 | 0.005 | 0.005 | 0.005 | 0.005 |
| Chloramphenicol | 0.05 | - | - | - | - |
| Dichloran | 0.002 | 0.002 | 0.002 | 0.002 | 0.002 |
| Bromocresol purple | 0.05 | 0.05 | 0.05 | 0.05 | 0.05 |
| Water to fill | 1000 | 1000 | 1000 | 1000 | 1000 |

Compositions were prepared similarly to FFA of Example 1 and autoclaved for 15 min at 121 °C.

### Example 7 - comparison of the growth of moulds on the growth media of the invention (FFA) with the growth medium according to prior art (MEA)

In this example, the growth of various mould-forming microorganisms was tested on the different media of the invention and compared to the reference medium MEA.

### Methods

FFAs according to the invention were prepared according to Table 3 and Example 6, while MEA was prepared according to Example 2.

A range of mould-forming microorganisms was tested, including filamentous fungi, yeasts with mould properties, and other fungi as reported in table 4.

**Table 4. Mould-forming microorganisms tested in Examples 7 and 8.**

| # | **Scientific name** | **Strain** |
|---|---|---|
| | Filamentous fungi | |
| 1 | *Penicillium crustosum* | DSM 62837 |
| 2 | *Penicillium roqueforti* | DSM 1079 |
| 3 | *Penicillium roqueforti* | P.roq FR |
| 4 | *Penicillium commune* | DSM 2211 |
| 5 | *Penicillium expansum* | DSM 1282 |
| 6 | *Penicillium Paneum* | CBS 101032 |
| 7 | *Penicillium Palitans* | CBS 111834 |
| 8 | *Penicillium chrysogenum* | DSM 4154 |
| 9 | *Penicillium verrucosum* | DSM 12639 |
| 10 | *Aspergillus niger* | DSM 11167 |
| 11 | *Aureobasidium pullulans var. pullulans* | M01 |
| 12 | *Fusarium solani* | DSM 1164 |
| 13 | *Fusarium oxysporum* | DSM 62057 |
| 14 | *Fusarium* | Fusarium |
| 15 | *Rizopus microsporus var. oligosporus* | CBS 228.95 |
| 16 | *Mucor racemosus* | DSM 5266 |
| 17 | *Mucor circinelloides f. circinelloides* | M02 |
| 18 | *Cladosporium allicinum* | DSM 27591 |
| 19 | *Cladosporioides* | Clado |
| 20 | *Epicoccum nigrum* | Phoma |
| 21 | *Epicoccum nigrum* | DSM891 |
| 22 | *Alternaria radicina* | CBS 118382 |

| | Taxonomically classified as yeasts, but harbor certain phenotypical traits of filamentous fungi | |
|---|---|---|
| 23 | *Geotrichum candidum* | DSM 1240 |
| 24 | *Trichosporon* | Trichosporon |

Spore solutions were prepared as explained in Example 2. Diluted spore solutions were placed on a petri dish containing either one FFA of Table 3 or MEA. Of each mould, all petri dishes were inoculated with the same spore solution and are therefore considered to contain the same amount of spores. The dishes were incubated at 25 °C for 5-10 days.

### Results

The results of growth are shown in Table 5.

**Table 5. Growth of mould-forming microorganisms on the different FFA media compared to the reference medium MEA. "++" = higher or comparable growth to MEA, "+" = slightly lower growth than on MEA, "-" = no growth, "/" = test not performed.**

| # | **Species** | **Strain** | **FFA30** | **FFA-Alkaline** | **FFA10** | **FFA-Nitrate** | **FFA-Ammonium** |
|---|---|---|---|---|---|---|---|
| Filamentous fungi | | | | | | | |
| 1 | *Penicillium crustosum* | DSM 62837 | ++ | + | ++ | ++ | ++ |
| 2 | *Penicillium roqueforti* | DSM 1079 | ++ | ++ | ++ | ++ | ++ |
| 3 | *Penicillium roqueforti* | P.roq FR | ++ | + | ++ | ++ | ++ |
| 5 | *Penicillium expansum* | DSM 1282 | ++ | ++ | ++ | ++ | ++ |
| 6 | *Penicillium Paneum* | CBS 101032 | + | - | - | ++ | ++ |
| 7 | *Penicillium Palitans* | CBS 111834 | ++ | + | ++ | ++ | ++ |
| 8 | *Penicillium chrysogenum* | DSM 4154 | ++ | + | - | ++ | ++ |
| 9 | *Penicillium verrucosum* | DSM 12639 | ++ | ++ | ++ | ++ | + |
| 10 | *Aspergillus niger* | DSM 11167 | + | - | + | + | ++ |
| 11 | *Aureobasidium pullulans var. pullulans* | M01 | ++ | + | ++ | ++ | ++ |
| 12 | *Fusarium solani* | DSM 1164 | - | / | - | ++ | ++ |
| 13 | *Fusarium oxysporum* | DSM 62057 | - | / | - | ++ | + |
| 14 | *Fusarium* | Fusarium | - | / | - | ++ | + |
| 15 | *Rizopus microsporus var. oligosporus* | CBS 228.95 | - | / | - | - | ++ |
| 16 | *Mucor racemosus* | DSM 5266 | - | / | - | + | ++ |
| 17 | *Mucor circinelloides f. circinelloides* | M02 | - | / | ++ | + | - |
| 18 | *Cladosporium allicinum* | DSM 27591 | ++ | / | ++ | ++ | ++ |
| 19 | *Cladosporioides* | Clado | ++ | / | ++ | ++ | + |
| 20 | *Epicoccum nigrum* | Phoma | ++ | / | ++ | ++ | ++ |
| 21 | *Epicoccum nigrum* | DSM891 | ++ | / | ++ | ++ | ++ |
| 22 | *Alternaria radicina* | CBS 118382 | ++ | / | ++ | ++ | ++ |

| Yeasts with filamentous fungi properties | | | | | | | |
|---|---|---|---|---|---|---|---|
| *23* | *Geotrichum candidum* | DSM 1240 | - | / | - | - | ++ |
| *24* | *Trichosporon* | Trichosporo n | - | / | - | - | - |

The results shown in Table 5 highlight the innate ability of a certain species to grow on a given FFA medium. All filamentous fungi and other fungi grew satisfactorily on all FFA media tested. Growth of yeast with mould like properties species was inhibited on the FFA media. This result indicates that the media of the invention are selective for the growth of mould due to fungi.

### Example 8 - growth of moulds and yeast on various FFA recipes

In this example, the growth of the mould-forming microorganisms of Example 7 in the presence of yeast was tested on the FFA media of the invention and compared to control medium MEA.

### Methods

FFA media, MEA, and spore solutions were prepared according to the preceding Examples.

Yeast culture of Saccharomycotina of approximately 2×10⁸ cfu/ml was used to make a serial dilution until 2×10³ cfu/ml. Spores from each mould of the fungi of table 4 were harvested. Each of the yeast dilutions was mixed with the spore solution of 4×10³ cfu/ml at a ratio of 0.9 ml yeast dilution and 0.1 ml spore. Thus, a final solution was created of 4×10² cfu/ml spores and 1.8×10⁸ cfu/ml yeast (where necessary the undiluted 2×10⁸ cfu/ml yeast was used). The resulting samples are reported in table 6. A log difference of 0 means no yeast present, values > 0 represent log factors of yeast competition: a log value of 1 represents a 10-fold excess of yeast, a log value of 2 100 fold excess of yeast, and so on.

**Table 6. Samples of spores and yeast. Concentrations are in cfu/ml.**

| **Sample** | **Spores** | **Yeast** | **Log difference** |
|---|---|---|---|
| 0 | 4×10² | 0 | - |
| 1 | 4×10² | 1.8×10³ | 1 |
| 2 | 4×10² | 1.8×10⁴ | 2 |
| 3 | 4×10² | 1.8×10⁵ | 3 |
| 4 | 4×10² | 1.8×10⁶ | 4 |
| 5 | 4×10² | 1.8×10⁷ | 5 |
| 6 | 4×10² | 1.8×10⁸ | 6 |

The samples were incubated on one of the FFA of table 3 or on MEA as described in Example 2. After incubation, the colony forming units (CFUs) of mould were counted.

### Results

Results are shown in Figure 6 and Table 7.

**Table 7. Lowest log difference between mould and yeast at which qualitative mould enumeration was still possible. "-" = not included in the analysis (e.g. no growth of mould), "/" = test not executed.**

| # | **Species** | **Strain** | **FFA30** | **FFA-Alkaline** | **FFA10** | **FFA-Nitrate** | **FFA-Ammonium** | **MEA** |
|---|---|---|---|---|---|---|---|---|
| Filamentous fungi | | | | | | | | |
| 1 | *Penicillium crustosum* | DSM 62837 | 6.0 | 6.0 | 6.0 | 6.0 | 6.0 | 1.5 |
| *2* | *Penicillium roqueforti* | DSM 1079 | 6.0 | 5.3 | 5.3 | 6.0 | 6.0 | 1.5 |
| *3* | *Penicillium roqueforti* | P.roq FR | 6.0 | 6.0 | 5.3 | 6.0 | 6.0 | 2.0 |
| *5* | *Penicillium expansum* | DSM 1282 | 6.0 | 6.0 | 6.0 | 6.0 | 6.0 | 3.0 |
| *6* | *Penicillium Paneum* | CBS 101032 | 6.0 | - | - | 5.7 | 2.0 | 1.5 |
| 7 | *Penicillium Palitans* | CBS 111834 | 6.0 | 6.0 | 6.0 | 6.0 | 5.3 | 4.0 |
| 8 | *Penicillium chrysogenum* | DSM 4154 | 2.0 | 2.0 | - | 6.0 | 6.0 | 2.0 |
| *9* | *Penicillium verrucosum* | DSM 12639 | 5.5 | 4.7 | 5.5 | 6.0 | 3.0 | 2.0 |
| 10 | *Aspergillus niger* | DSM 11167 | 5.0 | - | 4.0 | 6.0 | 6.0 | 4.0 |
| 11 | *Aureobasidiu m pullulans var. pullulans* | M01 | 6.0 | 6.0 | 6.0 | 6.0 | 6.0 | 5.0 |
| *12* | *Fusarium solani* | DSM 1164 | - | / | - | 6.0 | 4.7 | 1.0 |
| 13 | *Fusarium oxysporum* | DSM 62057 | - | / | - | 6.0 | 2.0 | 1.5 |
| 14 | *Fusarium* | Fusariu m | - | / | - | 6.0 | 1.5 | 1.0 |
| 15 | *Rizopus microsporus var. oligosporus* | CBS 228.95 | - | / | - | - | 1.0 | 2.0 |
| 16 | *Mucor racemosus* | DSM 5266 | - | / | - | 2.5 | 0.5 | 0.0 |
| 17 | *Mucor circinelloides f circinelloides* | M02 | - | / | 0.5 | 0.5 | - | 6.0 |
| 18 | *Cladosporiu m allicinum* | DSM 27591 | 6.0 | / | 6.0 | 6.0 | 3.7 | 2.0 |
| 19 | *Cladosporioi des* | Clado | 5.7 | / | 5.7 | 6.0 | 4.3 | 2.0 |
| 20 | *Epicoccum nigrum* | Phoma | 1.5 | / | 2.0 | 6.0 | 1.0 | 1.5 |
| 21 | *Epicoccum nigrum* | DSM89 1 | 4.5 | / | 3.3 | 6.0 | 6.0 | 3.0 |
| 22 | *Alternaria radicina* | CBS 118382 | 6.0 | / | 6.0 | 5.3 | 4.0 | 3.5 |

| Yeasts with filamentous fungi properties | | | | | | | | |
|---|---|---|---|---|---|---|---|---|
| 23 | *Geotrichum candidum* | DSM 1240 | / | / | - | - | 1.5 | 2.0 |
| 24 | *Trichosporon* | Trichos poron | / | / | - | - | - | - |
| | | Average | 5.2 | 5.3 | 4.8 | 5.5 | 3.9 | 2.4 |
| | | St. dev | 1.5 | 1.4 | 1.7 | 1.4 | 2.1 | 1.4 |

Table 7 indicates the log excess of yeast compared to mould at which the CFUs of mould could still be counted. A value of 6 indicates that at 1,000,000-folds yeast excess, CFUs of mould could be qualitatively counted. On average, it was possible to count CFUs of mould on all FFA recipes at higher excess of yeast than for MEA. On MEA it was possible to count CFUs of mould only up to around 100-fold excess of yeast, while on all FFAs it was possible to count CFUs of mould up to around 10,000-fold or 100,000-fold excess of yeast.

As it can be seen from Figure 6, it was found that detection accuracy of mould of filamentous fungi is significantly reduced on the control growth medium MEA when yeast levels are high. In contrast, it was possible to count CFUs of filamentous fungi on all tested FFA recipes, even at high yeast concentrations. FFA-Nitrate performed the best out of all FFA recipes.

Taken together these results indicated that all tested FFA recipes are better compared to the reference medium MEA in the detection of filamentous fungi at high levels of yeast. FFA-Nitrate showed the best discrimination power in the presence of high yeast levels.

### Example 9 - test with different yeasts

In this example, growth of different yeasts and *Penicillium roqueforti* is tested on the different FFA recipes and compared to MEA and two commercially available media known for the detection of mould: Czapek Dox and Synphony. Relative growth and colonies size is measured.

### Methods

The different FFA agar were prepared as in Example 6, the MEA was prepared as in Example 2. Czapek Dox and Symphony were bought/prepared. Their composition is reported in Table 8.

**Table 8. Composition of Czapek Dox and Symphony. All amounts are in g per 1 L of medium.**

| **Component** | **Amount** |
|---|---|
| **Czapek Dox** | |
| Sucrose | 30.0 |
| Sodium nitrate | 2.0 |
| Dipotassium phosphate | 1.0 |
| Magnesium sulphate | 0.50 |
| Potassium chloride | 0.50 |
| Ferrous sulphate | 0.010 |
| Agar | 15.0 |

| **Symphony** | |
|---|---|
| Peptones | 10.0 |
| Glucose | 18.0 |
| Growth promotors | 1.0 |
| Selective system | 1.0 |
| Bacteriological agar | 15.5 |

5 Yeast, 1 Filamentous fungus and 1 brine sample were prepared (Table 9). The yeast were grown on an MEA plate. One colony was resuspended in 10 ml ME-broth. A grown mould agar was used to harvest spores. 4 ml of Ringers with 0.05% Tween was used to collect the spores. After resuspending/collection of spores, the culture was counted under the microscope using a Thoma counting chamber. Based on the concentration the culture was diluted to reach a concentration of 300-500 cfu/ml. 0.1 ml of this concentration was plated on the various FFA and MEA. The brine sample was diluted until -5 in duplicate. 0.1 ml of each dilution was plated on the various FFA and MEA. Plates were incubated similarly to preceding Examples. After incubation, CFUs were counted, and the average of triplicates was determined.

**Table 9. Samples in Example 9.**

| Sample | Genus | Species | Strain |
|---|---|---|---|
| **Yeast** | | | |
| 1 | *Kluveromyces* | *Kluyveromyces lactis* | DSM 3795 |
| 2 | *Rhodotorula* | *Rhodotorula mucilaginosa* | Y03 |
| 3 | *Rhodoturulaspora* | | Rhodo1 |
| 4 | *Debaryomyces* | *Debaryomyces Hansenii* | LAF3 |
| 5 | *Wickerhamiella* | *Wickerhamiella versatilis* | DSM 6960 |

| **Filamentous fungi** | | | |
|---|---|---|---|
| 1 | *Penicillium* | *Penicillium roqueforti* | DSM 1079 |

| **Real sample** | | | |
|---|---|---|---|
| Brine | Brine | Brine | |

### Results

A good reproducibility between the replicates was observed.

The number of colonies per agar type were used to estimate the recovery in which the reference medium (MEA) was set to 100% (Table 10).

Surprisingly, yeast recovery is quite good in all agar variants tested (Table 10). Nonetheless a difference can be observed in terms of colony size, where MEA shows the largest colony sizes compared to the other recipes (Table 10 & Table 11). This suggests suboptimal growth for yeast on the FFA recipes comprising pectin.

**Table 10. Counts of yeast on different agar types, counts of MEA is set as 100%.**

| | **MEA** | **FFA 30** | **FFA-Alkaline** | **FFA 10** | **FFA-Nitrate** | **FFA-Ammonium** | **Czapek Dox** | **Symphon y** |
|---|---|---|---|---|---|---|---|---|
| *Kluveromyces* | 100% | 0% | 0% | 0% | 0% | 0% | 0% | 0% |
| *Rhodotorula* | 100% | 103 % | - | 140 % | 201% | 100% | 126% | 75% |
| *Rhodoturulasp ora* | 100% | 100 % | 264% | 127 % | 200% | 59% | 155% | 9% |
| *Debaryomyces* | 100% | 93% | 82% | 103 % | 96% | 128% | 112% | 0% |
| *Wickerhamiell a* | - | - | - | - | - | - | - | - |
| *Penicillium* | 100% | 115 % | 114% | 117 % | 106% | 116% | 65% | 9% |
| Brine | 100% | 69% | 5% | 13% | 133% | 118% | 11% | 0% |
| Average yeasts | 100% | 73% | 88% | 77% | 126% | 81% | 81% | 17% |

**Table 11. Relative size estimate of colonies on each agar. Size on MEA is set as 100%.**

| | **MEA** | **FFA 30** | **FFA-Alkaline** | **FFA 10** | **FFA-Nitrate** | **FFA-Ammonium** | **Czapek Dox** | **Symphon y** |
|---|---|---|---|---|---|---|---|---|
| *Kluveromyces* | 100% | 0% | 0% | 0% | 0% | 0% | 0% | 0% |
| *Rhodotorula* | 100% | 40% | - | 20% | 10% | 20% | 10% | 80% |
| *Rhodoturulasp ora* | 100% | 40% | 20% | 40% | 20% | 20% | 10% | - |
| *Debaryomyces* | 100% | 20% | 20% | 20% | 20% | 20% | - | 0% |
| *Wickerhamiell a* | *-* | - | - | - | - | - | - | - |
| *Penicillium* | 100% | 80% | 20% | 60% | 80% | 80% | 80% | 80% |
| Brine | 100% | 30% | 20% | 20% | 20% | 40% | 40% | 0% |
| Average yeasts | 100% | 26% | 15% | 20% | 14% | 20% | 15% | 20% |

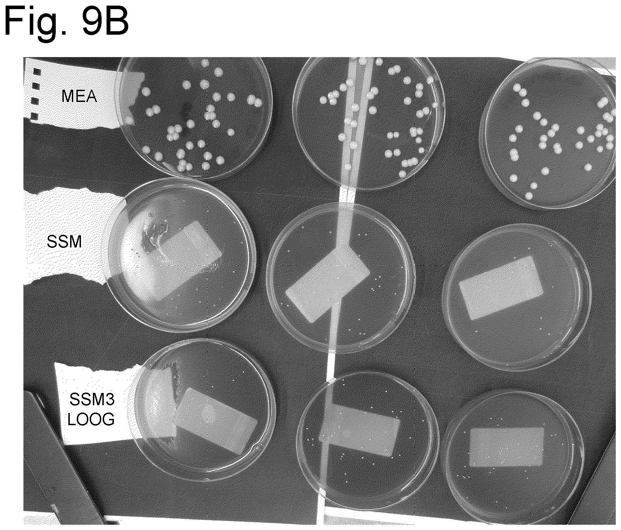

The results are also reported in Figure 9. As it can be seen in Figure 9, a similar number of colonies of yeast can be observed on MEA and FFA media. However, it is immediately clear that the colony size is much smaller of the FFA media than on MEA.

The performance of FFA media of the invention in terms of colony size is on average comparable to the commercially available media for the detection of yeast, i.e. Czapek Dox and Symphony (Table 11). Nevertheless, the performance of FFA media in terms of colony size is better than Czapek Dox and Symphony for the yeast strains *Rhodotorula.* Moreover, limited growth of the *Penicillium* and brine sample were observed on Czapek Dox and Symphony (Table 10), thus limiting their application in the field of food stuff where *Penicillium* is a common contaminant and/or brine is used, for example cheese making.

These results indicate that the FFA media effectively inhibit yeast growth by limiting colony size of yeast. FFA media are also better compared to commercially available recipes in the detection of mould of Penicillium or in brine samples. Thus, the FFA media are more suited to quality control in food production.

### Example 10 - Effective amount of pectin

In this example, different concentrations of pectin in FFA were tested to assess the optimal concentration for mould detection.

### Methods

In order to obtain eight different pectin concentrations (ranging from 0.00 to 0.5 g/L), 2 × 2L of FFA (Fungal Filamentous Agar) without pectin was prepared, using the following recipe:
Agar Medium Composition (per liter):

0.5 g KCl, 1 g K₂HPO₄, 0.5 g MgSO₄ × 7 H₂O, 6 g Sodium Nitrate (NaNO₃), 0.05 g Bromocresol purple, 0.002 g Dichloran, 0.01 g ZnSO₄ × 7H₂O, 0.005 g CuSO₄ × 5H₂O, 20 g Agar, 1000 ml Deionized Water, Adjust pH to 6.0 (+/- 0.1) and autoclave at 121°C for 15 minutes.

To create the desired pectin concentrations, pectin was added gradually, until the target pectin concentration was reached. Then 500 ml of medium was transferred to a 500 ml autoclave bottle. pH was always set to 6.0 (+/- 0.1) before transferring the agar.

This resulted in eight different agar types, each corresponding to a specific concentration expressed in grams of pectin per liter: FFA1 (0.00 g/L), FFA2 (0.01 g/L), FFA3 (0.1 g/L), FFA4 (0.5 g/L), FFA5 (1 g/L), FFA6 (5 g/L), FFA7 (10 g/L), and FFA8 (50 g/L).

The following steps were carried out for inoculation:
- Start with a yeast culture at a concentration of 1e8 cfu/ml and dilute it to 5e7 cfu/ml.
- Obtain an agar plate with *P. roqueforti,* allow it to grow, and collect spores using a Ringer's solution containing 0.05% Tween.
- Dilute the spore solution to a concentration of 4e3 cfu/ml.
- Take 1 ml of the spore solution and dilute it in 9 ml of Ringer's solution to create a pure spore solution.
- Take 1 ml of the spore solution and dilute it in 9 ml of the 5e7 cfu/ml yeast solution, creating a challenge solution.
- Inoculate 100 µl of the pure spore solution on each of the eight agar types in triplicate.
- Inoculate 100 µl of the challenge solution on each of the eight agar types in triplicate.
- Incubate the inoculated agar plates at 25°C for further observation.

### Results

The production of pectin medium was successful for all concentrations except FFA8, which contained 50 g/L of pectin . The FFA8 medium did not harden after 24 hours.

Figure 7 shows the growth of samples comprising i) spores of filamentous fungi only or ii) spores of filamentous fungi and yeast.

The sample comprising spores of filamentous fungi only yielded a significant number of colonies on the growth medium without pectin (0 g/L). At any pectin concentration higher than zero, the number of colonies observed was significantly higher (Figure 8A). There was no statistical difference between the 6 pectin concentrations of 0.01 g/L up to 10 g/L (Figure 8A).

The sample comprising both filamentous fungi and yeast did not yield any colonies at any pectin concentration below 0.5 g/L. The CFUs of mould increased with pectin concentration (Figure 7). There was no statistical difference between a pectin concentration of 0.5 g/L or 1g/L or between the concentrations of 5 g/L and 10 g/L (Figure 8B).

These data indicate that filamentous fungi easily grow on medium comprising pectin when the sample comprises only filamentous fungi. In the presence of yeast, the CFUs of mould can be counted optimally on growth media comprising pectin at a concentration of 0,5 wt.% or higher. Pectin concentrations above 10 wt.% were not achievable due to processing difficulties.

## Claims

1. A selective growth medium composition comprising carbohydrates, wherein the carbohydrates comprise pectin and agar, preferably wherein pectin and agar are the sole carbohydrates of the growth medium, wherein the selective growth medium is selective for the growth of filamentous fungi.

2. The growth medium composition according to claim 1, comprising pectin in an amount of 0.05 - 10 wt.% based on total weight of the composition.

3. The growth medium composition according to claim 1 or 2, wherein the amount of pectin and agar exceeds the amount of any monosaccharide or disaccharide by 50% based on the total weight of monosaccharides and disaccharides.

4. The growth medium composition according to any one of the preceding claims, wherein the pectin to agar weight ratio is in the range of 25:1 to 1:90, preferably 10:1 to 1:60, more preferably 10:1 to 1:10, most preferably 6:1 to 1:1.

5. The growth medium composition according to any one of the preceding claims, wherein the growth medium is for the detection of filamentous fungi, preferably wherein the growth medium is selective for the growth of filamentous fungi.

6. The growth medium composition according to any one of the preceding claims, wherein the composition further comprises a nitrogen source, wherein preferably the nitrogen source is creatine, at least an ammonium salt, at least a nitrate salt, or combinations thereof, more preferably at least a nitrate salt.

7. The growth medium composition according to any one of the preceding claims, wherein the growth medium composition is a gel.

8. Use of the gel growth medium composition according to claim 7, for quality control.

9. Use according to claim 8, wherein quality control is of food preparation processes and/or of air conditioning of buildings.

10. Use of the gel growth medium composition according to claim 7, for detecting filamentous fungi in a sample, wherein the sample preferably further comprises yeast.

11. A method for detecting filamentous fungi in a sample, wherein the method comprises
a. providing the sample to a container, preferably a petri dish, comprising the gel growth medium composition of claim 7;
b. incubating the container at a temperature of 10 to 40 °C, preferably 20 to 30 °C, more preferably 23 to 28 °C for at least 1 day up to at most 18 days;
c. counting the colony forming units (CFU) of mould.

12. The use according to claim 10 or the method according to claim 11, wherein the sample further comprises yeast, preferably wherein the yeast is selected from the subdivision *Saccharomycotina,* more preferably from *Ascoidea, Babjeviella, Brettanomyces, Candida, Clavispora, Cyberlindnera, Debaryomyces, Diutina, Eremothecium, Hyphopichia, Kazachstania, Kluyveromyces, Komagataella, Kuraishia, Lachancea, Lodderomyces, Metschnikowia, Meyerozyma, Naumovozyma, Ogataea, Pichia, Saccharomyces, Saccharomycodes, Saprochaete, Scheffersomyces, Spathaspora, Sugiyamaella, Suhomyces, Tetrapisispora, Torulaspora, Wickerhamiella, Wickerhamomyces, Yamadazyma, Yarrowia, Zygosaccharomyces, Zygotorulaspora* or combinations thereof.

13. The use according to claim 10 or the method according to claim 11, wherein the sample is selected from brine, a food product, or air, preferably wherein the sample is a food product, wherein the food product is selected from milk, cheese, and yogurt.

14. The growth medium composition of claim 5, the use of claim 10 or the method according to claim 11, wherein the filamentous fungi is selected from the genus *Penicillium* and/or *Aspergillus,* more preferably from *Aspergillus niger, Geotrichum candidum, Penicillium chrysogenum, Penicillium crustosum, Penicillium expansum, Penicillium verrucosum, Penicillium roqueforti, Penicillium commune,* or combinations thereof.

15. A kit of parts comprising
i. powdered carbohydrate, wherein the powdered carbohydrate comprises
a. powdered pectin, and
b. powdered agar;
ii. optionally at least one container; and
iii. an instructions manual;
wherein pectin and agar are the sole carbohydrates.
